(19) Europäisches Patentamt
European Patent Office
Office européen des brevets

(11) **EP 2 799 097 B1**

(12) **EUROPÄISCHE PATENTSCHRIFT**

(45) Veröffentlichungstag und Bekanntmachung des Hinweises auf die Patenterteilung:
**28.03.2018 Patentblatt 2018/13**

(51) Int Cl.:
***A61M 1/16*** *(2006.01)*

(21) Anmeldenummer: **14165194.3**

(22) Anmeldetag: **17.04.2014**

(54) **Vorrichtung zur extrakorporalen Blutbehandlung**

Device for treating blood outside the body

Dispositif destiné au traitement extracorporel du sang

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.05.2013 DE 102013104501**

(43) Veröffentlichungstag der Anmeldung:
**05.11.2014 Patentblatt 2014/45**

(73) Patentinhaber: **B. Braun Avitum AG**
**34212 Melsungen (DE)**

(72) Erfinder:
• **Dr. Strohhöfer, Christof**
**34123 Kassel (DE)**
• **Krause, Silvie**
**34212 Melsungen (DE)**
• **Ritter, Kai-Uwe**
**91126 Rednitzhembach (DE)**
• **Meibaum, Jörn**
**34225 Baunatal (DE)**

(74) Vertreter: **Winter, Brandl, Fürniss, Hübner, Röss, Kaiser, Polte - Partnerschaft mbB Patent- und Rechtsanwaltskanzlei Alois-Steinecker-Strasse 22 85354 Freising (DE)**

(56) Entgegenhaltungen:
EP-A2- 0 898 974   WO-A1-97/44072
WO-A2-00/38761   FR-A1- 2 801 794
US-A1- 2011 144 459

EP 2 799 097 B1

**Beschreibung**

Die Erfindung bezieht sich auf eine Vorrichtung zur extrakorporalen Blutbehandlung und insbesondere, aber nicht ausschließlich auf eine Vorrichtung nach dem Oberbegriff von Anspruch 1.

Bei Patienten mit eingeschränkter oder fehlender Nierenfunktion werden Abfallprodukte des natürlichen Stoffwechsels einschließlich urämischer Toxine durch Nierenersatz- bzw. Dialyseverfahren entfernt. Dabei erfolgt die Entfernung der Stoffe aus dem Blut, welches dem Patienten entnommen und extrakorporal geführt wird, durch den Kontakt des Blutes mit einer Dialysierflüssigkeit, wobei Blut und Dialysierflüssigkeit nicht direkt, sondern über eine semipermeable Membran miteinander in Kontakt stehen. Die Dialysierflüssigkeit ist mit verschiedenen Salzen versetzt. Die Entfernung der physiologischen Abfallprodukte erfolgt über diffusive und konvektive Effekte. Diese sind für den Stofftransport vom Blut in die Dialysierflüssigkeit über die extrakorporal angeordnete Membran verantwortlich. Nach erfolgter Entfernung eines Teils der Abfallstoffe wird das so behandelte Blut dem Patienten wieder zugeführt.

Zur Bewertung der Effizienz einer Dialysesitzung werden die Konzentrationen urämischer Toxine vor, nach und gegebenenfalls auch während einer Dialysesitzung bestimmt. Die Reduktion der jeweiligen Stoffe stellt dabei die zentrale Basis zur Bewertung der Dialysedosis dar.

Ein gängiger Leitmetabolit, der zur Bestimmung der Dialysedosis herangezogen wird, ist Harnstoff, auch Urea genannt. Entsprechend gilt die Harnstoffreduktionsrate als entscheidender Parameter in der Dialysetechnik. Die Bestimmung der Harnstoffreduktion kann auf unterschiedlichen Wegen erfolgen.

Eine klassische Methode stellt die chemische Bestimmung der Harnstoffkonzentration im Blut jeweils vor und nach einer Dialysetherapie dar. Das Problem dieser Methode ist jedoch, dass die Blutproben dem Patienten entnommen und zu einem Labor geschickt werden müssen, welches für die Bestimmung der Harnstoffkonzentration entsprechend ausgerüstet ist. Dieser Vorgang kann durchaus mehrere Tage in Anspruch nehmen. Die Bestimmung der Dialysedosis kann dementsprechend nicht zeitnah und vor allem nicht während einer Dialysesitzung erfolgen.

Eine andere Möglichkeit zur Bestimmung der Dialysedosis ist die Messung der UV-Absorption im Abfluss der Dialysierflüssigkeit. Uhlin hat in seiner Doktorarbeit mit dem Thema "Haemodialysis Treatment monitored on-line by ultra violet absorbance", Linköping University Medical Dissertation No 962, 2006, gezeigt, dass die Änderung des dekadischen Absorptionsmaßes in der abfließenden Dialysierflüssigkeit bei einer Wellenlänge von 280 nm eine sehr gute Korrelation zur Konzentrationsänderung von Harnstoff im Blut des Patienten aufweist. Diese Ergebnisse wurden kürzlich von Calia et al. bestätigt (Monitoring Urea, Creatinine and ß2-Microglobulin Concentrations in Spent Dialysate by Spectrophotometric and Spectrofluorimetric Measurements, Calia D., Di Francesco F., Fuoco R., Ghimenti S., Kanaki A., Onor M., Tognotti D., Donadio C., 52nd National Congress of the Italian Society of Nephrology, Genova, 21 - 24 September 2011).

Darüber hinaus sind im Stand der Technik die folgenden Vorrichtungen und Verfahren bekannt.

In der US 2011/0144459 A1 wird ein Detektor zum Erfassen der Konzentration einer Flüssigkeit bei einem Blutwäschegerät beschrieben, bei dem das Blut extrakorporal gereinigt wird. Der Detektor hat einen optischen Strahler, der Licht in die Flüssigkeit einstrahlt, einen optischen Detektor, der das Licht des Strahlers hinter der Flüssigkeit aufnimmt, und einen Detektor, der die einfallende Lichtintensität erfasst.

In der WO 00/38761 A2 wird die Bestimmung des Verteilungsvolumens eines Blutinhaltsstoffes während einer extrakorporalen Blutbehandlung beschrieben. Dabei wird die Konzentration eines Blutinhaltsstoffes stromauf zum Dialysator geändert, die Änderung stromab zum Dialysator gemessen und das Verteilungsvolumen des Blutinhaltsstoffes aus den Änderungen bestimmt.

In der EP 1 342 479 A1 wird schließlich eine Vorrichtung zum Erfassen und Einstellen des Flusses einer Dialyselösung in einer Hämodiafiltration beschrieben. Zwei Pumpen werden über den Einlasszweig und Auslasszweig des Dialyse-Kreislaufs verteilt. Ein Differentialflussmessgerät erfasst den Stromunterschied der Dialyse-Lösung in den Filter hinein und aus ihm heraus. Eine Zentralsteuerung erfasst Ausgangssignale des Differentialflussmessgerätes. Damit wird der Regeldruck der Dialyse-Lösung im Filter periodisch nachgeführt und der Strom der Dialyse-Lösung und des Plasma-Wassers durch die Membran des Filters eingestellt.

Die WO 97/44072 A1 offenbart eine Hämodialysemaschine und ein Verfahren für die online erfolgende Echtzeitüberwachung der Wirksamkeit der Hämodialysebehandlung und im spezielleren für die Schaffung eines Zwischenkammer-Transferkoeffizienten für die Zwei-Pool-Kinetik bei der Hämodialyse.

Das Hämodialyseüberwachungssystem quantifiziert die Rate und die Menge an Harnstoff, der während der Hämodialysebehandlung abgeführt wird, durch Messen der Harnstoffkonzentration in dem verbrauchten Austrittsdialysat in Abhängigkeit von der Zeit.

Von der Dialysataustrittsleitung aus der Hämodialysemaschine werden periodisch Proben entnommen, um ein kleines Volumen des verbrauchten Austrittsdialysats zu entnehmen, wenn eine ausreichende Fluidströmung erfaßt wird.

Die EP 0 898 974 A2 offenbart ein Verfahren zur Bestimmung von Parametern der Hämodialyse, das eine dialysatseitige Messung der Konzentration einer am Stoffaustausch des Dialysators teilnehmenden Substanz im Blut als der zu bestimmende Parameter oder als Zwischenwert für den zu bestimmenden Parameter während der extrakorporalen Blutbehandlung erlaubt.

Zusätzlich offenbart die EP 0 898 974 eine Blutbehandlungsvorrichtung mit einer Einrichtung zur Bestimmung von Parametern der Hämodialyse, die eine, dialysatseitige Messung einer am Stoffaustausch des Dialysators teilnehmenden Substanz im Blut als der zu bestimmende Parameter oder als Zwischenwert für den zu bestimmenden Parameter während der extrakorporalen Blutbehandlung erlaubt.

Die FR 2 801 794 A1 offenbart eine Methode zur kontinuierlichen Ermittlung eines Parameters (D, Cbin, K, Kt/V) in einer extracorporalen Blutbehandlundsvorrichtung (Dialysemaschine). Die Methode besteht unter anderem darin, daß ein mathematisches Modell benutzt wird, welches die Beziehung zwischen dem Wert Cdin der Ionenkonzentration (oder der Leitfähigkeit) einer Probe der Dialyseflüssigkeit stromauf des Dialysators und einem Wert Cdout der Ionenkonzentration (oder der Leitfähigkeit) einer Probe der Dialysierflüssigkeit stromabwärts des Dialysators, ausdrückt.

Problematisch bei dem genannten Stand der Technik sind das hohe Untergrundrauschen und der flache Anstieg des Messsignals zur Konzentrationsbestimmung von aus dem Blut zu reinigenden physiologischen Abfallprodukten.

Aufgabe der Erfindung ist es daher, eine Vorrichtung zur extrakorporealen Blutbehandlung der vorliegenden Gattung zu schaffen, mit der eine verbesserte Qualität des Messsignals für die Konzentrationsbestimmung bzw. die Bestimmung der Dialysedosis erreicht wird.

Diese Aufgabe wird erfindungsgemäß durch die Vorrichtung gemäß der unabhängigen Ansprüche gelöst. Vorteilhafte Weiterbildungen der Erfindung sind Gegenstand der Unteransprüche.

Die Erfindung geht von einer Messeinrichtung zur Bestimmung von Abfallprodukten in Dialyseflüssigkeiten während Dialysebehandlungen aus, wie sie insbesondere in der EP 1 083 948 B1 beschrieben ist, in welcher der Aufbau als auch die Position der bekannten Messvorrichtung (Sensors) für dialysetechnische Anwendungen erläutert werden. Die folgenden Ausführungen beziehen sich im Wesentlichen auf einen UV-Sensor in der Dialyse, wie in der EP 1 083 948 B1 beschrieben.

Für die laufende bzw. kontinuierliche Messung der Konzentration einer bestimmen Substanz bzw. einer Kombination von Substanzen, die in den in der austretenden Dialyseflüssigkeit enthaltenen Abfallprodukten enthalten ist/sind, ist in der EP 1 083 948 B1 an der Ausgangsleitung des Dialysators, hinter dem Dialysator innerhalb oder hinter der Dialysemaschine, eine transparente Meßzelle angebracht, welcher durch einen Lichtleiter UV-Licht von einer Lichtquelle zugeführt wird.

Das Licht, das der Meßzelle zugeführt und durch diese Meßzelle sowie die dadurch strömende Dialyseflüssigkeit hindurchgeht, wird absorbiert und von den in der Dialyseflüssigkeit vorhandenen Abfallprodukten in unterschiedlichem Maße gestreut, so daß eine erhöhte Konzentration einer Substanz in der Dialyseflüssigkeit die Lichtdurchlässigkeit reduziert. Das durchgeleitete Licht wird von einem Lichtleiter zu einem Photodetektor umgelenkt, um in diesem zu einem elektrischen Signal umgewandelt zu werden.

Derartige UV-Sensoren sind heutzutage in vielen modernen Dialysemaschinen integriert. Ihre Position ist seit der Markteinführung solcher Systeme im Wesentlichen unverändert geblieben. Sie befinden sich im Abfluss der Dialysierflüssigkeit, in der Regel der Bilanzierungseinrichtung nachgelagert, also stromab zu der Bilanzierungseinrichtung. Die Länge der dialysierflüssigkeitsführenden Leitungen/Schläuche zwischen dem Dialysator und der Messeinrichtung an dieser Position beträgt normalerweise mehr als 450cm, das gesamte Dialysierflüssigkeitsvolumen zwischen dem Dialysator und der Messeinrichtung beträgt ca. 200ml. Daneben sind weiter Sensorsysteme bekannt, wie sie beispielhaft in der DE 10 2011 008 482 A1 oder der EP 2 397 167 A1 beschrieben sind. In der DE 10 2011 008 482 A1 wird eine UV-Detektoranordnung mit einer Messküvette mit mindestens abschnittsweise UV-transparenter Wandung beschrieben, wobei zwei UV-LEDs mit unterschiedlichen Emissionswellenlängen, ein breitbandiger UV-Detektor bzw. zwei schmalbandige UV-Detektoren für jeweils eine der Emissionswellenlängen an der Messküvette angeordnet sind. In der EP 2 397 167 A1 wird eine Messeinrichtung zur Bestimmung der Lumineszenz der verbrauchten Dialysierflüssigkeit beschrieben. Die folgenden Ausführungen gelten für alle Sensorsysteme, welche ein Maß für die Konzentration urämischer Toxine in der verbrauchten Dialysierflüssigkeit liefern, analog.

Neben dem bisherigen Einsatz zur (quasi)statischen Bestimmung eines Maßes für die Konzentration urämischer Toxine tritt zunehmend die Quantifizierung von Transienten, d.h. zeitlicher Abläufe von Konzentrationsveränderungen, in den Vordergrund. Konzentrationstransienten geben Aufschluss über dynamische Patienten- und Maschinenparameter, deren Kenntnis es dem Arzt erlaubt, die Qualität der Therapie patientenindividuell zu erhöhen. Die derzeitige Positionierung der UV-Messeinrichtung mit einer langen fluidführenden Strecke bzw. mit einem hohen Volumen zwischen dem Dialysator und der Messeinrichtung bewirkt aber einige unvorteilhafte Effekte. So durchläuft die abfließende Dialysierflüssigkeit auf dem Weg zwischen Dialysator und UV-Messvorrichtung eine Reihe von fluidführenden Elementen, welche aufgrund ihres Volumens dazu beitragen, dass Konzentrationsänderungen nivelliert und zeitlich nicht mehr aufgelöst werden können. Es treten konvektive Mischeffekte auf, die die Transienten stark verändern, bis hin zu einer vollständigen Auflösung ihres Informationsgehalts. Bei dem resultierenden Messsignal lässt sich oftmals nicht mit Sicherheit unterscheiden, ob es sich z.B. um eine kurzzeitig auftretende Konzentrationsspitze des betreffenden Abfallproduktes handelt oder um eine länger anhaltende Erhöhung. Die Informationen über Patient und Therapie, die in den Transienten enthalten sind, gehen dabei verloren. Typische Elemente im Dialysierflüssigkeitsablauf einer Dialysemaschine, die dies bewirken, sind Bilanzierungseinrichtungen, z.B. Bilanzkammern, oder Luftabscheider.

Zum anderen kommt es zu einer signifikanten zeitlichen Verzögerung zwischen dem Auftreten einer Konzentration oder Konzentrationsänderung in der Dialysierflüssigkeit der dialysierflüssigkeitsseitigen Kammer des Dialysators und deren Nachweis mittels der Messvorrichtung. Bis zur Ankunft des Signals an der Messeinrichtung können gegebenenfalls mehrere Minuten vergehen. Diese Zeitdauer, während der verbrauchte Dialysierflüssigkeit vom Dialysator zur Messeinrichtung transportiert wird, führt dazu, dass sich Transienten auch durch Diffusion urämischer Toxine über die dort natürlicherweise existierenden Konzentrationsgradienten verändern. Auch hier kommt es zu einem Informationsverlust.

Während die Degeneration der Transienten aufgrund konvektiv wirkender Elemente dadurch verhindert werden kann, dass die Messeinrichtung zwischen Dialysator und den entsprechenden Elementen platziert wird, bedarf die Vermeidung der diffusiven Degeneration des Transientensignals weitergehender Überlegungen:

Harnstoff als ein typischer Repräsentant für kleinmolekulare urämische Toxine besitzt einen Diffusionskoeffizienten in wässriger Umgebung von $D = 1{,}38 \cdot 10^{-5}$ cm²/s, Kalium als ein typischer in Blut und Dialysierflüssigkeit vorhandener Elektrolyt einen darüber hinausgehenden von $D = 2 \cdot 10^{-5}$ cm²/s. Da im Schlauch des Dialysierflüssigkeitsabflusses die Reynoldszahl so dimensioniert ist, dass laminare Flussbedingungen vorherrschen, kann die Degeneration einer scharfen Transienten, die zum Beispiel aufgrund eines Ventilschaltvorgangs zustande kommt, mittels folgender Beziehung zwischen Diffusionszeit und Diffusionsweg, die aus den Fickschen Gesetzen abgeleitet ist, abgeschätzt werden:

$$x = 2\sqrt{Dt}$$

Hierbei steht D für den Diffusionskoeffizienten des betrachteten Stoffes im relevanten Trägermedium (hier: Dialysierflüssigkeit), t ist die Zeit, während derer die Diffusion abläuft und sich ein Diffusionsprofil entwickelt, und x ist eine Maßzahl für die Breite des entstehenden Diffusionsprofils.

Die Anwendung dieser Abschätzung auf die oben genannten Stoffe ergibt typische Diffusionswege von 300 - 350 μm für eine Diffusionszeit von 1 min. Dies führt bei typischen Dialysierflüssigkeitsflüssen und -schlauchdurchmessern zu einer Unschärfe im Signal auf der Skala von einigen Millisekunden, welches bereits im Bereich der Schaltzeiten von schnellen Ventilen liegt, und entsprechende Relevanz z.B. bei der Diskrimination von Artefakten aus Schaltvorgängen für Regelungen besitzt.

Die obige Bewertung des Diffusionseffekts wurde unter der Annahme einer konstanten Flussverteilung über den Querschnitt des Dialysierflüssigkeitskanals oder -schlauchs durchgeführt (sogenanntes Top-Hat-Profil). Diese Annahme wurde getätigt, um den Effekt der Diffusion isolieren zu können. In realen laminaren Flusssystemen liegt stattdessen ein parabolisches Profil der Form (B. J. Kirby, Micro- and Nanoscale Fluid Mechanics, Cambridge University Press, 2010)

$$u_z = -\frac{1}{4\eta}\frac{\partial \rho}{\partial z}(R^2 - r^2)$$

vor. Dabei bedeutet $u_z$ die Geschwindigkeit der Flüssigkeit entlang des Kanals oder Schlauches, $\eta$ die Viskosität der Flüssigkeit, $\dfrac{\partial \rho}{\partial z}$ ist der Druckgradient, R der Innendurchmesser des Kanals oder Schlauches, und r der radiale Abstand des betrachteten Punkts vom Zentrum des Kanals oder Schlauches. Die maximale Flussgeschwindigkeit, $u_z^{max} = -\dfrac{1}{4\eta}\dfrac{\partial \rho}{\partial z}R^2$ ergibt sich im Zentrum des Kanals oder Schlauchs für r = 0. Am Rand des Kanals oder Schlauchs gilt $u_z = 0$. Als Funktion der maximalen Flussgeschwindigkeit lässt sich das parabolische Flussprofil vereinfacht schreiben als

$$\frac{u_z}{u_z^{max}} = 1 - \left(\frac{r}{R}\right)^2$$

Dieses parabolische Flussprofil führt dazu, dass Konzentrationsänderungen, welche an einem Ort z = 0 entstehen, an einem Ort z > 0 erst nach einer endlichen Zeit t mit hinreichender Genauigkeit messbar sind.

$$t_0 = \frac{R^2 \pi}{Q}z,$$

Diese Zeit t ist größer als die Zeit welche sich ergäbe, wenn die Flüssigkeit über den gesamten Kanal- oder Schlauchquerschnitt dieselbe Geschwindigkeit aufweisen würde. Dabei ist Q der Volumenstrom (Fluss) des Mediums im Kanal oder Schlauch. Dieser auf das Flussprofil zurückzuführende Effekt beeinträchtigt die Güte von Messungen, welche innerhalb einer bestimmten Zeit nach Entstehen des Pulses durchgeführt werden müssen. Dies kann z.B. der Fall sein, wenn ein Regelalgorithmus in kurzen Zeitintervallen einen Datenwert benötigt oder wenn für die Messung nur ein kurzer Konzentrationspuls zur Verfügung steht.

Um dies zu verstehen, werden die folgenden Überlegungen vollzogen:

Die mittlere Flussgeschwindigkeit im Kanal oder Schlauch lässt sich ermitteln aus dem Volumenstrom der Flüssigkeit Q und der Geometrie:

$$\overline{u} = \frac{Q}{R^2\pi}$$

Zudem lässt sich aus dem parabolischen Flussprofil die mittlere Geschwindigkeit in Abhängigkeit der Maximalgeschwindigkeit bestimmen

$$\overline{u}_z = \frac{\int_0^R u_z(r)\cdot r\,dr}{\int_0^R r\,dr} = \frac{u_z^{max}}{2}$$

Damit lässt sich das Geschwindigkeitsprofil in Abhängigkeit des Volumenstroms anstatt der Maximal-geschwindigkeit schreiben

$$u_z(r) = 2\frac{Q}{\pi R^2}\left[1 - \left(\frac{r}{R}\right)^2\right]$$

An einem Ort z > 0 können nun zwei Zonen definiert werden: eine im Zentrum mit 0 < r < r', wo eine hohe Flussgeschwindigkeit herrscht und wohin bereits Flüssigkeit der neuen (c, hier im folgenden annahmegemäß der höheren) Konzentration transportiert worden ist, und eine zweite am Rand r' < r < R, wo noch Flüssigkeit der alten ($c_0$ im folgenden annahmegemäß Konzentrati-on 0) Konzentration vorhanden ist. Die Grenze zwischen diesen Bereichen bei r' kann ermittelt werden über

$$u_z(r') = 2\frac{Q}{\pi R^2}\left[1 - \left(\frac{r'}{R}\right)^2\right] = \frac{z}{t}$$

Aufgelöst nach $r'^2$ erhält man

$$r'^2 = 1 - \frac{1}{2}\frac{\pi R^2}{Q}\frac{z}{t}$$

Die mittlere Konzentration $\overline{cc}$ im Punkt z > 0 zum Zeitpunkt t > 0 ergibt sich als gewichtetes Mittel aus c und $c_0$:

$$\overline{c} = r'^2\pi\cdot c + \left(R^2 - r'^2\right)\pi\cdot c_0$$

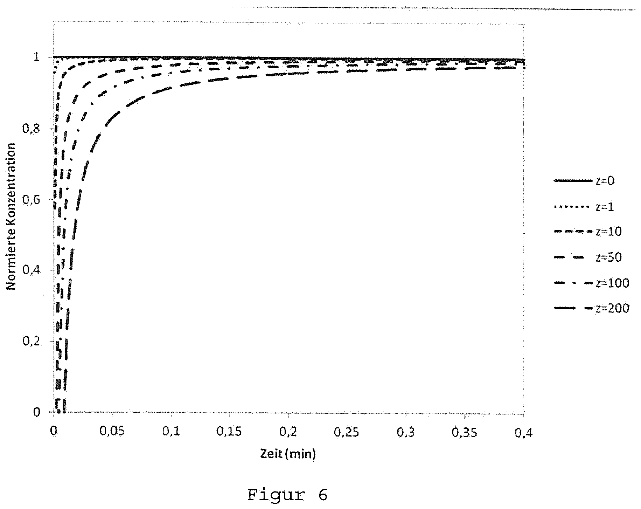

Figur 6 zeigt die Entwicklung der mittleren Konzentration über die Zeit für Messorte unterschiedlichen Abstands vom Entstehungsort eines Konzentrationssprungs bzw. einer Konzentrationsänderung. Die Konzentration springt zum Zeitpunkt t = 0 im Ort z = 0 von

$c_0$ = 0 auf c = 1. Der Schlauchdurchmesser wurde hier beispielhaft mit 0,6 cm angenommen, der Volumenfluss mit 300 ml/min. Die Abbildung zeigt deutlich, dass das laminare parabolische Flussprofil dazu führt, dass bereits bei einem Abstand von wenigen Dezimetern die Information über die Konzentration selbst bei Veranschlagung einer moderaten Genauigkeit von 5% erst nach mehreren Sekunden vorliegt.

Dies wird vor allem dann problematisch, wenn die zu bestimmende Konzentrationsänderung nur über einen begrenzten Zeitraum von wenigen Sekunden vorliegt (z.B. wie in EP 1 083 948 B1 beschrieben) oder wenn Information aus der Transienten des Signals erhalten werden soll.

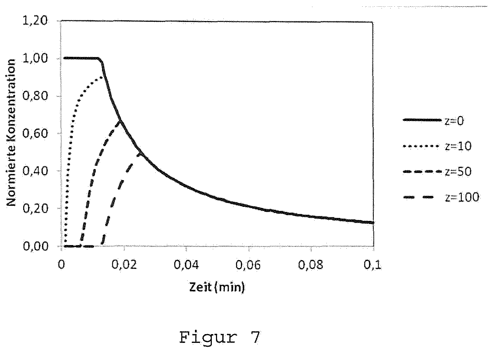

Dies wird aus Figur 7 deutlich. Hier ist für einen Volumenfluss von 100 ml/min und einer Pulslänge von 100 cm (Konzentration c = 1) die zeitliche Entwicklung der Konzentration bei verschiedenen Positionen der Messvorrichtung mit Abständen z > 0 dargestellt.

Sehr deutlich ist das Auswaschen der Flanken des Pulses zu sehen. Zudem wird die Konzentration c bereits bei Abständen von unter 10 cm nicht mehr erreicht.

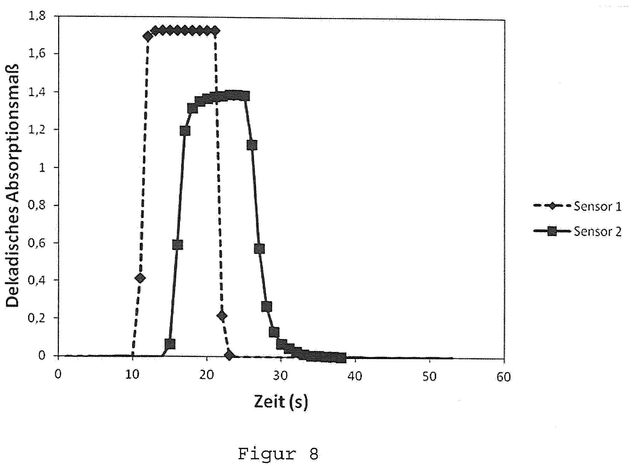

Die theoretischen Überlegungen gemäß der Figur 7 werden bestätigt durch experimentelle Messung der UV-Absorbanz, wie dies in Figur 8 dargestellt ist. Dabei wurde zum Zeitpunkt t = 10sec ein Bolus einer Harnsäurelösung von 40 mg/l in ein Dialyseschlauchsystem mit Dialysierflüssigkeitstfluss von 300 ml/min unmittelbar stromauf zu einem Sensor 1 injiziert. Harnsäure ist ein urämisches Toxin, das während der Dialyse entfernt wird. Die gewählte Konzentration entspricht einer typischen Konzentration in der Dialysierflüssigkeit während einer Dialyse. Die Bolusgabe dauerte 11sec. Der Sensor 1 sieht den Bolus als scharfen Peak im dekadischen Absorptionsmaß, welches direkt proportional zur Konzentration ist. Die Abbildung zeigt einen gut definierten Bolus, mit scharfen Flanken. Der Wert des dekadischen Absorptionsmaßes auf dem Plateau von etwa 1,7 entspricht der Absorption der Harnsäure. Stromab zum Sensor 1 passierten die Dialysierflüssigkeit (und der darin injizierte Konzentrationsbolus) ein Schlauchstück von 220 cm Länge, an dessen Ende ein zweiter UV-Sensor angebracht war. Das vom Sensor 2 gemessene dekadische Absorptionsmaß ist ebenfalls in Figur 8 dargestellt. Deutlich erkennt man

(a) eine Verzögerung der führenden Flanke des Bolus um 5sec, welche auf die Wegstrecke zwischen den Sensoren zurückzuführen ist. Ferner ergibt sich, dass

(b) das dekadische Absorptionsmaß sein Plateau erst 8sec nach Start des Bolusses gegenüber 1sec bei Sensor 1 erreicht. Quantitativ lässt sich feststellen, dass

(c) bei Sensor 2 das Plateau des dekadischen Absorptionsmaßes 20% unter dem bei Sensor 1 liegt - dies führt zu einem entsprechenden Fehler bei der Bestimmung der Konzentration. Schließlich lässt sich

(d) eine fallende Flanke im Signal von Sensor 2 von 7sec Länge identifizieren, im Vergleich zu der Flanke von 1sec Länge des gleichen Bolus in Sensor 1.

Figur 8 zeigt damit deutlich, dass sich die Signalqualität hinsichtlich des Erreichens des Plateaus und der Definition der Flanken eines Bolus im Experiment verschlechtert, je weiter der Sensor vom Entstehungsort des Bolus entfernt ist.

Aus Fig. 6, 7 und 8 wird klar, dass der Anstieg (die Schärfe) der Flanken und das (zeitnahe) Erreichen von c stark von der Position der Messvorrichtung abhängen. Sowohl die Qualität der führenden Flanke als auch der Fehler bei der Konzentrationsbestimmung werden kleiner, je näher sich die Messvorrichtung am Entstehungsort der Konzentrationsänderung (des Konzentrationssprungs) befindet.

Als Beispiel hierfür soll die Genauigkeit der Konzentrationsbestimmung dienen, welche sich bei der Zeit t = 0,1 min aus Figur 6 ergibt. Bei einem Abstand der Messvorrichtung von 10 cm vom Entstehungsort der Konzentrationsänderung wird unter den genannten Voraussetzungen die Konzentration c mit einem Fehler von unter 0,05% bestimmt. Dagegen beträgt bei einem Abstand von 200 cm der Fehler bereits 8,5%. Letztere Fehler sind für eine genaue Regelung von Therapieparametern bereits nicht mehr zu gebrauchen.

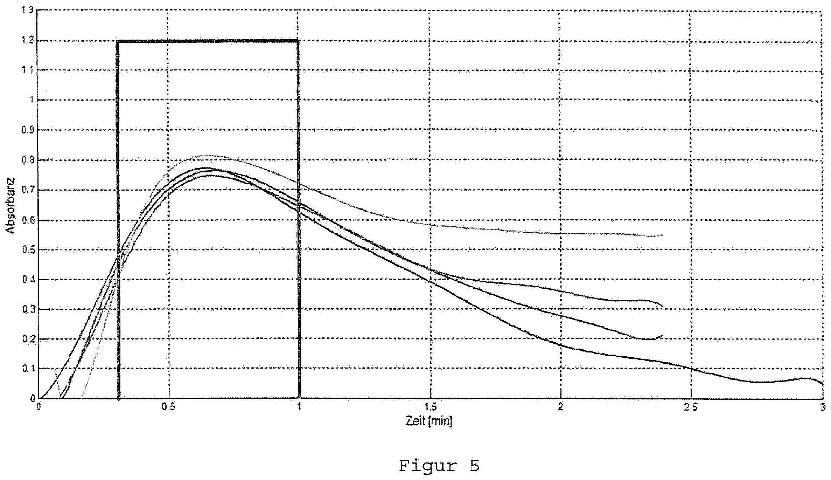

Figur 5 zeigt eine weitere experimentelle Untermauerung der vorstehend dargestellten Überlegungen. Hier wurde bei einem Dialysierflüssigkeitsfluss von 500 ml/min ein scharfer Konzentrationspuls einer Substanz von 300 ml in den Dialysator eingebracht. Die Abbildung zeigt die Antwort einer UV-Messvorrichtung, welche ca. 100 cm hinter dem Abfluss des Dialysators angebracht war. Das Experiment wurde mehrfach wiederholt und zeigt jedesmal ähnliche, der Darstellung in Figur 7 entsprechende Resultate, nämlich die bereits vor nominalem Eintreffen des Konzentrationspulses ansteigende Flanke, die langgezogene fallende Flanke, sowie eine Maximalkonzentration unterhalb von $c_0$. Die experimentellen Ergebnisse enthalten natürlich neben dem Einfluss des parabolischen Profils auf die Messgenauigkeit auch Effekte der Diffusion sowie etwaige zusätzliche konvektive Effekte durch Querschnittsvergrößerungen/-verkleinerungen im Kanal oder Schlauch, die in ihrer Gesamtheit die für die Messung abträglichen Effekte vergrößern.

In der Dialyse ist der Ort der Entstehung einer Konzentrationsänderung oder eines Konzentrationspulses meist der Dialysator. Entsprechend besteht eine Lösung des beschriebenen Problems gemäß der Erfindung darin, die Messvorrichtung als eine Bedingung höchstens 250 cm, vorzugsweise weniger als 200 cm, vorzugsweise weniger als 150 cm, vorzugsweise weniger als 100 cm, vorzugsweise weniger als 50 cm, vorzugsweise weniger als 20 cm Schlauch- oder Kanallänge vom Abfluss der verbrauchten Dialysierflüssigkeit aus dem Dialysator zu platzieren.

Obige Rechnungen wurden durchgeführt unter der Annahme von Kanälen und/oder Schläuchen gleichbleibenden Durchmessers. In Dialysemaschinen ist dies jedoch nicht unbedingt gewährleistet. Aufgrund von Variationen im Kanal- oder Schlauchdurchmesser, verursacht z.B. von Koppelstücken, Ventilen und sonstigen fluidischen Elementen, und den damit verbundenen Änderungen in der Flussgeschwindigkeit bei konstantem Volumenfluss, kann der vorstehende Lösungsansatz bestimmter Schlauchlängen analog auf Volumina zwischen Entstehungsstelle einer Konzentrationsänderung und Position der Messvorrichtung als eine zweite zusätzliche oder alternative Bedingung übertragen werden. Eine vorteilhafte Positionierung des Sensors sollte gemäß dieser zweiten Bedingung erfindungsgemäß kleiner gleich 150 ml (Leitungs- und Komponenten-Füllvolumen) vom Abfluss der verbrauchten Dialysierflüssigkeit aus dem Dialysator bis zum Sensor erfolgen, vorzugsweise kleiner gleich 100 ml, vorzugsweise kleiner gleich 50 ml, vorzugsweise kleiner gleich 35 ml, vorzugsweise kleiner gleich 20 ml bei einem (mittleren) Kanal-/Schlauchquerschnitt von vorzugsweise ca. 3-7mm (weiter vorzugsweise ca. 5mm) und unter Berücksichtigung der Dialysierflüssigkeitsvolumen der im Dialysierflüssigkeitspfad angebrachten Komponenten.

Auf der Grundlage der vorstehenden theoretischen und analytischen Betrachtungen wurde gefunden, dass eine Anordnung der Detektions-/Messvorrichtung (Sensor) am proximalen (d.h. Dialysator nahen) Abschnitt des Abflusses der Dialysierflüssigkeit aus der dialysierflüssigkeitsseitigen Kammer eines Filterelements unter Erfüllung zumindest einer der zwei Bedingungen das gestellte Problem löst und die zuvor beschriebenen Probleme im Stand der Technik überwindet.

Die erfindungsgemäße Vorrichtung zur extrakorporalen Blutbehandlung hat unter anderem vorzugsweise die folgenden Bauteile:

- ein Filterelement (Dialysator), das in eine blutseitige Kammer und in eine dialysierflüssigkeitsseitige Kammer geteilt ist mit einem Zufluss für eine frische Dialysierflüssigkeit zur dialysierflüssigkeitsseitigen Kammer und einem Abfluss für die verbrauchte Dialysierflüssigkeit aus der dialysierflüssigkeitsseitigen Kammer,

- eine Bypassleitung,

- eine Detektionsvorrichtung, die im Betrieb kontinuierlich von aus dem Abfluss abfliessender Dialysierflüssigkeit durchflossen wird und dazu ausgelegt ist, die zeitliche Konzentrationsänderung mindestens eines Abbauprodukts aus dem Blut des Patienten dialysierflüssigkeitsseitig zu erfassen und diese erfassten Werte bereit zu stellen, wobei

- die Detektionsvorrichtung an einer Position stromab zum Abfluss der Dialysierflüssigkeit aus der dialysierflüssigkeitsseitigen Kammer des Filterelements direkt an der Fluidleitung angebracht ist, wobei

- die Detektionsvorrichtung zwischen dem Abfluss der Dialysierflüssigkeit aus der dialysierflüssigkeitsseitigen Kammer des Filterelements und einem Anschluss der Bypass Leitung angeordnet ist und das sich ergebende Fluidleitungs- und/oder Komponenten - Füllvolumen zwischen dem Abfluss und der Detektionsvorrichtung kleiner oder gleich 100 ml ist,
- eine dem Filterelement nachgeschaltete Bilanzierungseinrichtung,
- ein Gehäuse, innerhalb welchem unter anderem zumindest die Bilanzierungseinrichtung und vorzugsweise die Detektions-/Messvorrichtung eingehaust sind und außerhalb welchem das Filterelement angeordnet ist sowie eine Fluidleitungsdurchführung durch das Gehäuse, über die das Filterelement mit der Bilanzierungseinrichtung fluidverbunden/fluidverbindbar ist.

Dabei kann die erfindungsgemäße Vorrichtung zur extrakorporalen Blutbehandlung zudem weitere Bauteile umfassen, die aus dem Stand der Technik per se bekannt sind und zur Standardausstattung moderner Dialysemaschinen gehören oder gehören werden, wie beispielsweise eine Druckmessvorrichtung vorzugsweise in/an der Gehäusewand im Bereich der Fluidleitungsdurchführung und damit unmittelbar stormauf zur Detektions-/Messvorrichtung und/oder eine das Filterelement kurzschließende Bypassleitung vorzugsweise stromab zur Detektions-/Messvorrichtung oder alternativ stromauf zur Detektions-/Messvorrichtung, dann u.a. zum Zweck einer Kalibrierung der Detektions-/Messvorrichtung. Da diese weiteren Bauteile aber für die vorliegende Erfindung nur von sekundärer Bedeutung sind, werden diese der Übersichtlichkeit halber vorliegend nicht im Detail angeführt.

Die erfindungsgemäße Vorrichtung zur extrakorporalen Blutbehandlung kann dabei jedwede Geräte umfassen, die sich zur Dialyse eignen, insbesondere künstliche Nieren und Dialysemaschinen. Vorteilhaft an der Erfindung ist die Verbesserung der Messgenauigkeit und Schärfe des Messsignals zur Konzentrationsbestimmung von aus dem Blut zu reinigenden physiologischen Abfallprodukten. Die zuvor geschilderten Probleme bei der extrakorporalen Blutbehandlung werden somit gelöst und das Erkennen und Quantifizieren von Konzentrationsänderungen wird mit besonderer Genauigkeit ermöglicht.

Weitere Merkmale und Vorteile der Erfindung ergeben sich aus der folgenden Beschreibung, bei der Bezug genommen wird auf die beigefügte Zeichnung.

Figur 1 zeigt einen Dialysierflüssigkeitskreislauf mit einer Vorrichtung zur extrakorporalen Blutbehandlung für eine Hämodyalyse,

Figur 2 zeigt einen Dialysierflüssigkeitskreislauf mit einer Vorrichtung zur extrakorporalen Blutbehandlung für eine Hämodiafiltration,

Figur 3 zeigt einen Dialysierflüssigkeitskreislauf mit einer Vorrichtung zur extrakorporalen Blutbehandlung zum Entzug von Flüssigkeit während einer sequentiellen Therapie

Figur 4 zeigt einen Dialysierflüssigkeitskreislauf mit einer Vorrichtung zur extrakorporalen Blutbehandlung für eine Hämofiltration,

Figur 5 zeigt, wie bereits beschrieben, die Antwort einer UV-Messvorrichtung an einer Position nach dem Stand der Technik,

Figur 6 zeigt, wie bereits beschrieben, den Verlauf der Konzentration über die Zeit bei verschiedenen Positionen der Messvorrichtung nach einer sprunghaften Konzentrationsänderung,

Figur 7 zeigt, wie bereits beschrieben, die Antwort einer UV-Messvorrichtung in Abhängigkeit ihrer Positionierung auf einen Konzentrationspuls bei der erfindungsgemäßen Vorrichtung,

Figur 8 zeigt, wie bereits beschrieben, die UV-Absorbanz nach einem Bolus, wie sie von einem ersten und einem zweiten Sensor erfasst wird,

Figur 9 zeigt das Messergebnis einer Dialysemaschine mit einer UV-Messvorrichtung an einer herkömmlichen Position und

Figur 10 zeigt einen Vergleich der Reaktionsverzögerung auf einen Konzentrationspuls von Harnsäure in einer Dialysemaschine mit einer UV-Messvorrichtung an einer herkömmlichen Position und in einem proximalen (Dialysator nahen) Abschnitt des Abflusses der Dialysierflüssigkeit.

Die Figuren sind rein illustrativ und daher nicht maßstäblich. Gleiche oder gleich wirkende Elemente sind mit denselben Bezugszeichen versehen, soweit nichts anderes gesagt ist.

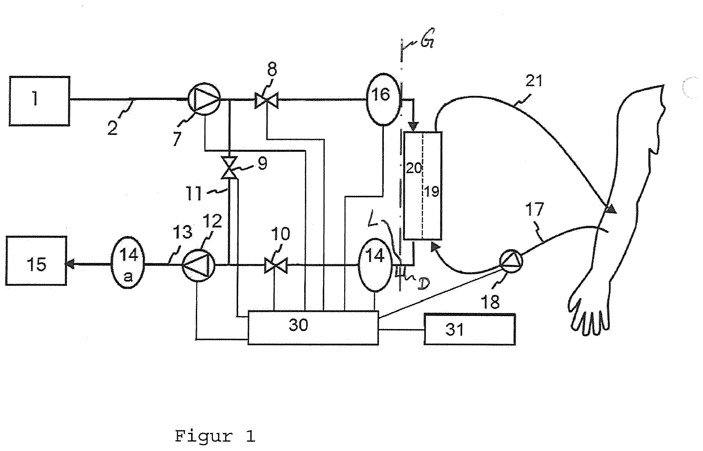

Figur 1 zeigt eine erste Ausführungsform des erfindungsgemäßen Dialysierflüssigkeitskreislaufs in einer Vorrichtung zur extrakorporalen Blutbehandlung (z.B. Dialysemaschine). Die Vorrichtung wird hier zur Durchführung einer Hämodialyse ohne Substitution von Flüssigkeit eingesetzt. Frische Dialysierflüssigkeit wird durch eine Dialysierflüssigkeitsquelle 1 bereitgestellt und über einen Zufluss 2 in den Kreislauf gespeist. Dazu ist eine Flusspumpe (FPE) 7 stromauf zu einem Filterelement (Dialysator) vorgesehen. Ein erstes Ventil (VDE) 8 zwischen der Flusspumpe 7 und dem Filterelement verschließt bzw. öffnet einen Eingang des Filterelements.

Ein zweites Ventil (VDA) 10 stromab zum Filterelement öffnet bzw. schließt den Dialysierflüssigkeitskreislauf zu einem Ausgang des Filterelements. Analog zur Eingangsseite befindet sich auch auf der Ausgangsseite des Filterelements eine Flusspumpe 12, welche die verbrauchte Dialysierflüssigkeit zum Dialysierflüssigkeitsausgang fördert, von wo es über eine Dialysierflüssigkeitsabflussleitung 13 zu einem Dialysierflüssigkeitsabfluss 15 abgeführt/entsorgt wird. Die eingangsseitige Flusspumpe 7 und die ausgangsseitige Flusspumpe 12 sind über eine Bypass-Leitung 11 miteinander fluidverbunden, welche so in den Dialysierflüssigkeitskreislauf angeschlossen ist, dass sie das Filterelement wahlweise kurzschließt. Hierfür ist Bypass-Leitung 11 durch ein Bypass-Ventil (VBP) 9 verschließbar oder öffenbar, wobei sie zudem stromauf zum ersten Ventil 8 sowie stromab zum zweiten Ventil 10 angeordnet ist.

Erfindungsgemäß befindet sich eine Detektions-/Messvorrichtung 14 nahe am Filterelement (Dialysator), d.h. zwischen dem Ausgang des Filterelements und der Bypass - Leitung 11, vorzugsweise zwischen dem Filterelementausgang und dem zweiten Ventil 10. Ergänzend oder alternativ in einer nicht beanspruchten Variante, befindet sich eine Detektions-/Messvorrichtung 14a stromab zu der Bypass - Leitung 11.

An dieser Stelle sei darauf hingewiesen, dass bei der vorliegenden Vorrichtung zur extrakorporalen Blutbehandlung das Filterelement außerhalb eines in der Figur 1 durch eine Strich-Punkt-Line dargestellten Gehäuses G befindet, wohingegen die Detektionsvorrichtung 14, 14a im Gehäuse G eingehaust ist. In/an der Gehäusewand ist optional ein Drucksensor D vorzugsweise stromauf zur Detektionsvorrichtung 14, 14a angeordnet. Weiter vorzugsweise ist die Detektionsvorrichtung 14 unmittelbar an der Gehäusewand, d.h. unmittelbar an einer Leitungsdurchführung L platziert, welche den gehäuseäußeren Leitungsabschnitt ausgehend vom Filterelementausgang mit dem gehäuseinneren Leitungsabschnitt fluidverbindet. Auf diese Weise ist die Detektionsvorrichtung 14 innerhalb des Gehäuses geschützt und trotzdem leitungs-/strömungstechnisch möglichst nahe dem Filterelement angeordnet.

In der Regel werden Leitungen (Schläuche) mit einem Leitungsinnenquerschnitt von ca. 3 - 7 mm, vorzugsweise 5 mm verwendet. Erfindungsgemäß ist eine vorteilhafte Positionierung der Detektionsvorrichtung 14 oder 14a (d.h. des Sensors) dann gegeben, wenn das Schlauch-/Leitungsvolumen zwischen dem Filterelementausgang und der Detektionsvorrichtung 14 oder 14a kleiner gleich 100 ml, vorzugsweise kleiner gleich 50 ml, vorzugsweise kleiner gleich 35 ml, vorzugsweise kleiner gleich 30 ml, vorzugsweise kleiner gleich 15 ml und weiter vorzugsweise kleiner gleich 7 ml beträgt, ebenfalls jeweils vorzugsweise bei einem Strömungsquerschnitt von ca. 3 - 7 mm (ca. 5 mm). Alternativ oder zusätzlich hierzu beträgt eine Leitungslänge zwischen dem Filterelementausgang und der Detektionsvorrichtung 14 oder 14a höchstens 250 cm, vorzugsweise weniger als 200 cm, vorzugsweise weniger als 150 cm, vorzugsweise weniger als 100 cm, vorzugsweise weniger als 50 cm und weiter vorzugsweise weniger als 20 cm Schlauch- oder Kanallänge. Dabei sei darauf hingewiesen, dass in Abhängigkeit vom Modell der Dialysemaschine bei einem Volumen von weniger als 20-30 ml bzw. bei einer Schlauchlänge von weniger als 100 cm eine Positionierung des Sensors außerhalb des Maschinengehäuses erfolgt, sodass die Einhausung des Sensors erstrebenswert aber nicht grundsätzlich immer verwirklichbar ist.

Mittels einer Flussmesseinrichtung 16 zwischen dem ersten Ventil 8 und dem Filterelement wird optional der im Kreislauf umgewälzte Fluidvolumenstrom bestimmt/gemessen.

Der Patient ist über ein arterielles Schlauchsystem 17 mit der Vorrichtung zur extrakorporalen Blutbehandlung (Dialysemaschine) verbindbar. Eine arterielle Blutpumpe (BPA) 18 ist dafür vorgesehen oder angepasst, dem Patienten ungereinigtes Blut zu entziehen und es einer Blutseite (BS) 19 im Filterelement zuzuführen, die mit einer Dialysierflüssigkeitsseite (DS) 20 im Filterelement über eine semipermeable Membran in Verbindung steht. Aus der Blutseite (BS) 19 im Filterelement kann dann gereinigtes Blut über ein venöses Schlauchsystem 21 wieder dem Patienten zugeführt werden.

Gesteuert wird der Kreislauf und die Komponenten für die dialysierflüssigkeitsseitige, wie auch die blutseitige Fluidumwälzung durch eine Recheneinheit 30, die mit einem User-Interface 31 versehen ist.

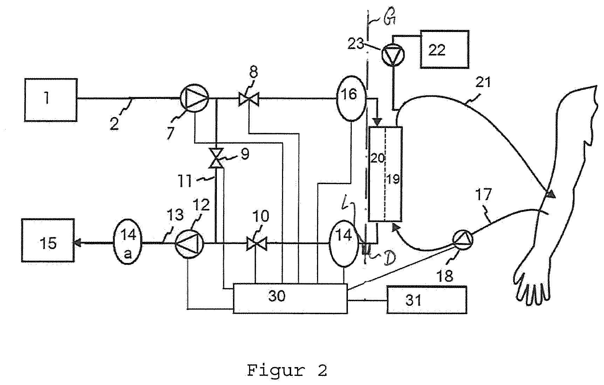

Fig. 2 zeigt eine weitere Ausführungsform des erfindungsgemäßen Dialysierflüssigkeitskreislaufs in einer Vorrichtung zur extrakorporalen Blutbehandlung. Wie bei der Ausführungsform nach Fig. 1, ist die Detektionsvorrichtung 14, 14a alternativ oder kombiniert an zwei verschiedenen Stellen im Abfluss der verbrauchten Dialysierflüssigkeit positionierbar, nämlich nahe am Filterelement (Dialysator) bei Position 14 zwischen Dialysator und Bypass 11 bzw. zweitem Ventil 10, vorzugsweise unmittelbar (vorzugsweise ohne Zwischenleitung) an (innen oder außen) der Leitungsdurchführung L durch das Gehäuse G bzw. ergänzend oder alternativ hinter der Bypass - Leitung 11 (bzw. Flusspumpe 12) bei Position 14a sofern im letzteren Fall die vorstehend genannten Bedingungen bezüglich maximaler Leitungslänge und/oder Leitungsvolumen erfüllt sind. Im Unterschied zu der Ausführungsform nach Fig. 1 zeigt Fig. 2 die Vorrichtung jedoch zur Durchführung einer Hämodiafiltration mit Substitution. Die Substitutionsflüssigkeit wird in diesem Fall auf der Blutseite stromab zu dem Dialysator mittels einer Substitutionspumpe 23 aus einem Substitutionsflüssigkeitsreservoir 22 in den Blutkreislauf infundiert. Dies führt zu konvektiver Reinigung des Bluts. Nicht dargestellt, aber analog zu realisieren, ist die Möglichkeit, die Substitutionsflüssigkeit blutseitig stromauf zu dem Dialysator in den Blutkreislauf zu infundieren bzw. sowohl stromauf als auch stromab zum Dialysator zu infundieren.

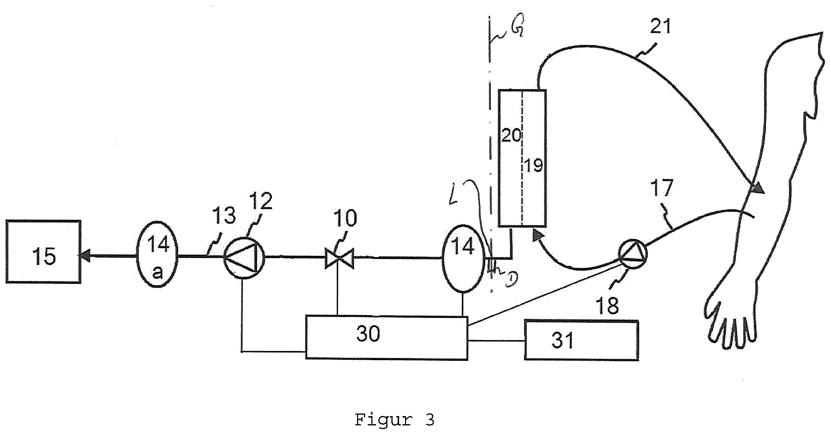

In Fig. 3 zeigt die Blutbehandlungsvorrichtung (Dialysemaschine) zur Durchführung einer sequentiellen Phase (reine Ultrafiltration) ohne Substitution von Flüssigkeit. Demnach ist insbesondere ein weiterer Dialysierflüssigkeitskreislauf gezeigt, bei dem die Detektionsvorrichtung 14, 14a alternativ oder in Kombination an zwei verschiedenen Stellen im Abfluss der verbrauchten Dialysierflüssigkeit angeordnet ist (sein kann).

Erfindungsgemäß befindet sich die Detektionsvorrichtung nahe am Dialysator (unmittelbar innen oder außen an der Leitungsdurchführung L in das Gehäuseinnere) bei Position 14 zwischen dem Dialysator und dem zweiten Ventil 10, und ergänzend oder alternativ befindet sich die Detektionsvorrichtung stromab zu dem Ventil 10 bei Position 14a (sofern die Bedingung bzgl. Leitungslänge und/oder Leitungsvolumen erfüllt ist).

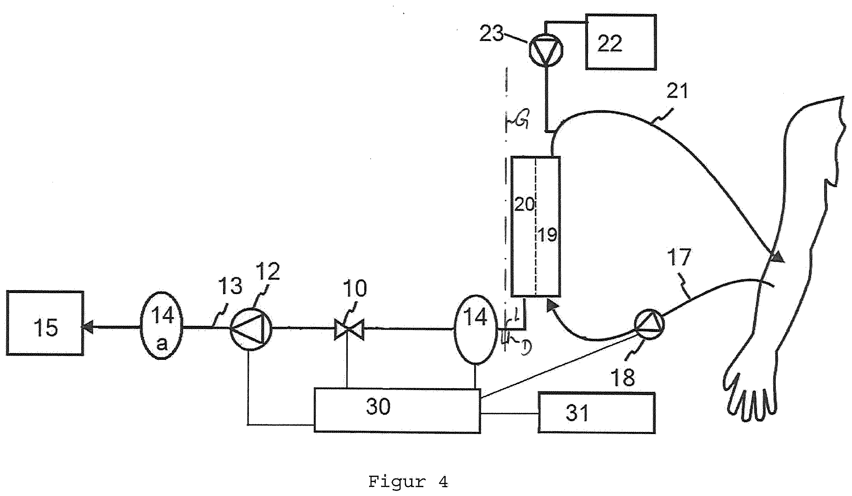

Figur 4 zeigt noch einen weiteren Dialysierflüssigkeitskreislauf in einer Vorrichtung zur extrakorporalen Blutbehandlung, die vorliegend für eine Hämofiltration eingesetzt wird, wonach die Detektionsvorrichtung 14, 14a alternativ oder in Kombination an zwei verschiedenen Stellen im Abfluss der verbrauchten Dialysierflüssigkeit positioniert ist, erfindungsgemäß nahe am Dialysator (unmittelbar an/nach der Leitungsdurchführung L durch das Gehäuse G) bei Position 14, und ergänzend bzw. alternativ stromab zu dem Ventil 10 bei Position 14a (unter Erfüllung der vorstehend genannten Bedingung bezgl. Leitungslänge und/oder Leitungsfüllvolumen). Substitutionsflüssigkeit wird hier mittels einer Substitutionspumpe 23 aus einem Substitutionsflüssigkeitsreservoir 22 blutseitig stromab und/oder stromauf zu dem Dialysator in den Blutkreislauf infundiert.

Figur 5 zeigt die Antwort der Detektionsvorrichtung vorliegend in Form einer UV-Messvorrichtung auf einen Konzentrationspuls am Filterelement, hier ein Dialysator, wenn die UV-Messvorrichtung nach dem Stand der Technik angeordnet ist. Gezeigt sind vier Messungen unter identischen Bedingungen, die die durch den Fluidweg von über 450cm bedingte Streuungsbreite der Systemantwort aufzeigen. Die Figur zeigt die Antwort des UV-Sensors auf einen Konzentrationspuls, welcher bei Abwesenheit der durch die Fluidführung bedingten Diffusions- und Konvektionseffekte als Rechteckpuls, wie in der Abbildung dargestellt, wahrgenommen werden sollte. Diffusionseffekte sowie das Flussprofil in den Leitungen/Schläuchen führen auch dazu, dass die vom UV-Sensor wahrgenommene Konzentration bereits vor der normalen Verzögerungszeit anzusteigen beginnt.

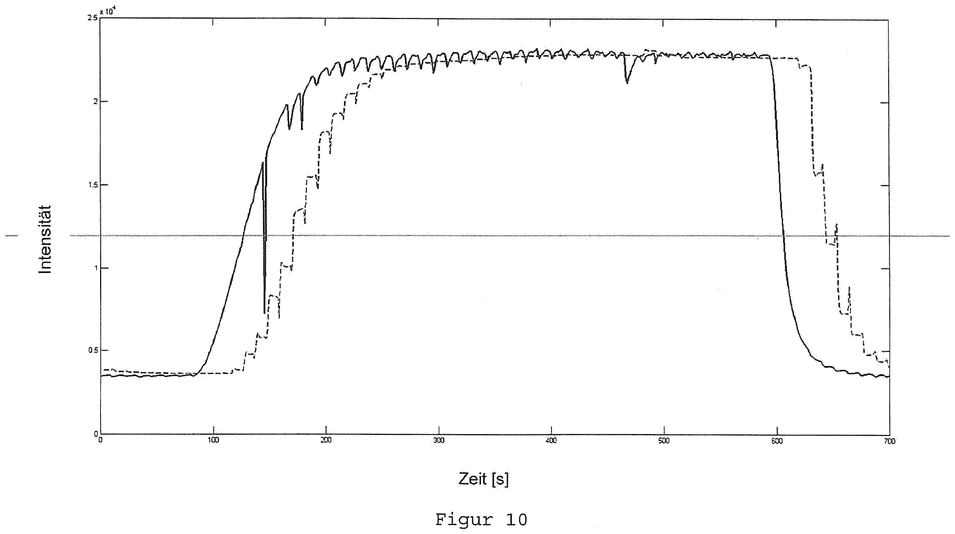

Figur 10 zeigt im Gegensatz hierzu die Antwort einer UV-Messvorrichtung auf einen Konzentrationspuls, wenn die UV-Messvorrichtung an einer solchen Stelle in der dialysierflüssigkeitsseitigen Stelle in der Abflussleitung stromab zum Filterelement angebracht ist, welche die vorstehenden Bedingungen bzgl. Leitungslänge und/oder Leitungsfüllvolumen nicht erfüllt (gestrichelte Linie), und die Antwort, wenn die UV-Messvorrichtung an einer Stelle in der dialysierflüssigkeitsseitigen Stelle in der Abflussleitung stromab zum Filterelement angebracht ist, welche die vorstehenden Bedingungen bzgl. Leitungslänge und/oder Leitungsfüllvolumen erfüllt (durchgezogene Linie). Die Messung an der die Bedingungen nicht erfüllenden Stelle (beispielsweise hinter der Bilanzierungseinrichtung) zeigt eine Verzögerung von etwa 30sec gegenüber der Messung an der die Bedingungen erfüllenden Stelle. Zudem weist die Messung an der die Bedingungen nicht erfüllenden Stelle stromab zu der Bilanzierungseinrichtung gemäß der Fig. 10 Stufen im Anstieg auf, welche in diesem Fall durch die Funktionsweise der vorgeschalteten Bilanzierungseinrichtung (mechanisches Umschalten) bestimmt sind. Diese vermindern die Signalqualität des Anstiegs und erschweren dessen Auswertung.

Optionale Ausgestaltungen der Erfindung

Die Erfindung ist nicht auf die oben beschriebenen Ausführungsformen beschränkt. Im Sinne der vorliegenden Erfindung ist vielmehr unter der die Bedingungen erfüllenden Stelle konstruktiv häufig derjenige Bereich des Abflusses der Dialysierflüssigkeit aus der dialysierflüssigkeitsseitigen Kammer des Filterelements zu verstehen, der sich unmittelbar an den Ausflussstutzen der dialysierflüssigkeitsseitigen Kammer des Filterelements anschließt jedoch bevorzugt noch innerhalb des Vorrichtungsgehäuses liegt.

Dieser Leitungsabschnitt kann sich somit je nach Dimensionierung der Blutbehandlungsvorrichtung sowie Platzierung des Filterelements maximal vom Ausflussstutzen der dialysierflüssigkeitsseitigen Kammer des Filterelements bis zum Einlass in die Bilanzierungseinrichtung erstrecken. Der Abschnitt kann ferner eine Spülbrücke umfassen. Die Detektionsvorrichtung kann sich bei einem solchen Aufbau stromauf oder stromab zu der Spülbrücke befinden; sie ist jedoch vorzugsweise in jedem Fall vor der Bilanzierungseinrichtung angeordnet, um eine Verfälschung und Verschlechterung des Signals verursacht durch die Bilanzierungseinrichtung zu verhindern. Obgleich die Detektionsvorrichtung bevorzugt innerhalb des Vorrichtungsgehäuses angeordnet ist, um diese gegen äußere Kräfte zu schützen, kann diese auch extern d.h. außerhalb des Gehäuses an die Vorrichtung zur extrakorporalen Blutbehandlung angebracht sein, insbesondere dann, wenn nur dadurch die vorstehend genannten Bedingungen bezgl. Leitungslänge und/oder Leitungsfüllvolumen erfüllbar sind. Unter dem Begriff "bis zur Detektionsvorrichtung" wird ferner die Stelle bezeichnet, wo in oder an der Detektionsvorrichtung die Messung vorgenommen wird. Dies ist vorzugsweise die Stelle, wo beispielsweise im Fall eines UV-Sensors der optische Messstrahl die Dialysierflüssigkeit durchquert.

Bevorzugt umfasst der Leitungsabschnitt zwischen Filterelement und Detektionsvorrichtung gemäß der obigen Festlegung maximal den Bereich vom Ausfluss des Filterelements bis zur Bilanzierungseinrichtung. Bevorzugt ist ein Bereich von 250 cm (Leitungslänge) in Flussrichtung von dem Auslass oder Abfluss des Filterelements in Richtung Bilanzierungseinrichtung , weiter bevorzugt 200 cm, noch weiter bevorzugt 150 cm, noch weiter bevorzugt 100 cm, noch weiter bevorzugt 90 cm, noch weiter bevorzugt 80 cm, noch weiter bevorzugt 70 cm, noch weiter bevorzugt 60 cm, noch weiter bevorzugt 50 cm, noch weiter bevorzugt 40 cm, noch weiter bevorzugt 30 cm, noch weiter bevorzugt 20 cm und am meisten bevorzugt 10 cm von dem Auslass oder Abfluss des Filterelements in Richtung Bilanzierungseinrichtung.

Weiter bevorzugt bezieht sich diese Anmeldung auf eine Vorrichtung zur extrakorporalen Blutbehandlung, bei der die Detektionsvorrichtung eine UV-Messvorrichtung ist. Diese umfasst zumindest eine Strahlungsquelle und zumindest einen Sensor. Als Strahlungsquellen kommen UV-LEDs, UV-Laser und breitbandige Strahlungsquellen wie z.B. Deuteriumlampen in Frage. Die Sensoren werden aus der Gruppe bestehend aus Photodioden, Phototransistoren, CCD- und CMOS-Detektoren, Photomultiplier oder Photon-Counting Modulen oder Elementen vergleichbarer Funktion ausgesucht. Ebenfalls ist bevorzugt, wenn der Sensor der UV-Messvorrichtung mindestens im Wellenlängenbereich von 200 bis 350 nm arbeitet. Dieser Wellenlängenbereich ist von besonderem Interesse, da in diesem Wellenlängenbereich viele für die Exkretion bedeutsame Metaboliten bzw. physiologische Abfallprodukte einen charakteristischen Absorptionspeak aufweisen, beispielsweise Harnsäure bei 290 nm, Kreatinin bei ca. 235 nm. Hippursäure bei ca. 260 nm und Kreatin bei ca. 210 nm. Aus einem solchen charakteristischen Absorptionspeak oder aus einer kombinatorischen Analyse mehrerer dieser Peaks lässt sich verlässlich auf die jeweilige Konzentration des Metaboliten zurückschließen.

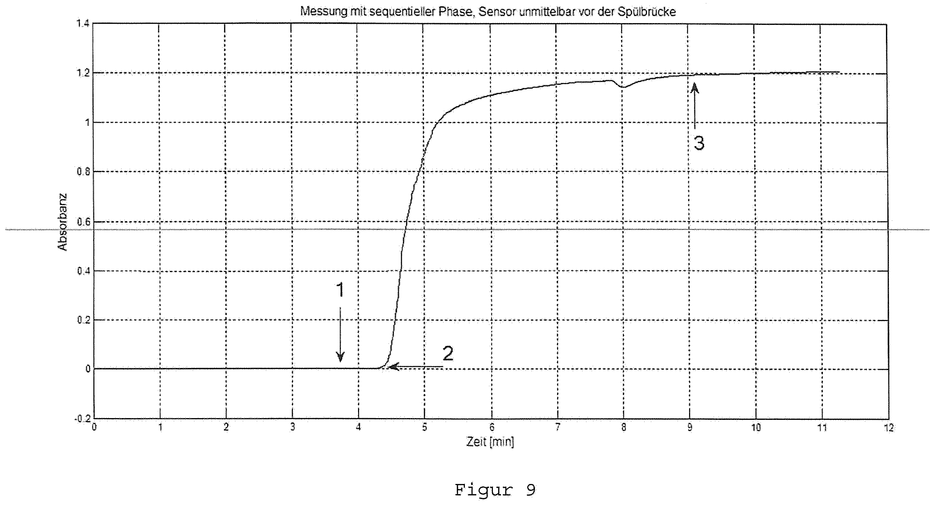

Im folgenden werden drei Beispiele angegeben, in denen der Unterschied zwischen dem Stand der Technik und der Erfindung deutlich wird. Dabei wird Bezug genommen auf das Messergebnis nach Fig. 9.

In einem ersten Beispiel aus dem Stand der Technik wird in einer Dialysemaschine mit einer UV-Messvorrichtung an einer herkömmlichen Position zum Zeitpunkt "1" am Dialysator die Konzentration von Harnsäure in herkömmlicher Dialysierflüssigkeit von null auf einen konstanten Wert (83,4 mg/l) erhöht, indem zum Zeitpunkt "1" die Maschine bei laufender Blutpumpe aus dem Bypass genommen wird. Der Fluss der Dialysierflüssigkeit beträgt konstant 500 ml/min, dies entspricht einer realistischen Flussgeschwindigkeit der Dialysierflüssigkeit bei einer Hämodialyse. Die Temperatur der Dialysierflüssigkeit am Punkt der UV-Messvorrichtung beträgt ca. 35 °C. Auch dies entspricht den üblichen Bedingungen einer Hämodialyse. Erst 30 - 40 Sekunden später treffen erste Harnsäuremoleküle am Sensor ein, d.h. zum Zeitpunkt "2". Bis sich das Signal vollständig entwickelt hat, vergehen mehr als 5 Minuten seit der Zugabe der Harnsäure, d.h. bis zum Zeitpunkt "3". Aus Figur 9 lässt sich zudem ersehen, dass der langsame Anstieg dem zuvor beschriebenen Vermischungseffekt geschuldet ist.

In einem zweiten Beispiel aus dem Stand der Technik wird in einer Dialysemaschine mit einer UV-Messvorrichtung an einer herkömmlicher Position ein zeitlich klar umrissener Konzentrationspuls von 45sec Länge appliziert, der in Figur 5 als Rechteckimpuls ersichtlich wird. Dieser Impuls wird mit Hilfe einer Ansättigung eines beschränkten klar abgegrenzten Volumens von Dialysierflüssigkeitt im Dialysator erreicht, welches anschließend ausgepumpt wird. Alle weiteren Versuchsbedingungen sind dieselben wie in dem ersten Beispiel oben. Als Folge dieses Konzentrationspulses wird die Absorption an der UV-Messvorrichtung für vier verschiedene Durchläufe gemessen. Das Signal verliert nach Durchlaufen der abflussseitigen Dialysierflüssigkeitsführung in der Dialysemaschine seine ursprüngliche Form und wäscht aus. Start- und Endpunkt des Pulses sind aus dem Signal der UV-Messvorrichtung nicht eindeutig zu bestimmen. Das Konzentrationsprofil wirkt stark verrundet, es entsteht eine lange abfallende Flanke am Ende des Pulses und eine etwas kürzere Flanke am Start des Pulses, welche beispielsweise durch Diffusionseffekte oder auf das Flussprofil zurückzuführende Effekte während des Wegs durch die Maschinenfluidik bedingt ist, wie dies bereits in der Beschreibungseinleitung ausgeführt wurde. Aufgrund des Vermischungseffekts, der abhängig ist von der Fluidstrecke zwischen Dialysator und UV-Messvorrichtung, leidet auch die Reproduzierbarkeit der Messwerte. Dies ist in Fig. 5 vor allem an der Qualität der Flanken des Konzentrationspulses, aber auch in der Höhe des Absorptionsmaximums zu erkennen. Bei vier Versuchen unter identischen Bedingungen trat eine nicht zu vernachlässigende Streuung der Ergebnisse auf.

In einem dritten Beispiel wird die Reaktionsverzögerung auf einen Konzentrationspuls von Harnsäure in einer Dialysemaschine mit einer UV-Messvorrichtung an einer herkömmlichen Position (im zentralen Abschnitt des Abflusses der Dialysierflüssigkeit der Bilanzierungseinrichtung in Strömungsrichtung nachgelagert) mit derjenigen mit einer UV-Messvorrichtung im proximalen Abschnitt des Abflusses der Dialysierflüssigkeit verglichen. Der Verlauf beider Kurven ist in Figur 10 zu sehen. Bei einer Anbringung der UV-Messvorrichtung in der erfindungsgemäßen Position ist der Konzentrationspuls signifikant früher zu detektieren. Wiederum ist der bereits bekannte Verzögerungseffekt zu erkennen. Das eigentlich glatte Signal weist nach der Bilanzierungseinrichtung aufgrund von Vermischungseffekten Stufen auf, wenn die Messung mit hoher zeitlicher Auflösung erfolgt. Hierzu tragen auch nicht-laminare bis turbulente Strömungsverhältnisse in und um die Bilanzierungseinrichtung bei.

Die Erfindung bezieht sich zusammenfassend auf eine Vorrichtung zur extrakorporalen Blutbehandlung, mit einer Detektionsvorrichtung (14) zur Erfassung urämischer Toxine in einer verbrauchten Dialysierflüssigkeit vorzugsweise durch Absorbanzmessung, wobei die Detektionsvorrichtung (14) an einer solchen Position stromab zum Abflusses (13) der Dialysierflüssigkeit aus einem Filterelement angebracht ist, die folgende Bedingungen erfüllt:

das Fluidleitungs-/Komponenten - Füllvolumen ausgehend vom Abfluss der verbrauchten Dialysierflüssigkeit aus dem Filterelement bis zur Detektionsvorrichtung (14) ist kleiner gleich 100 ml, vorzugsweise kleiner gleich 50 ml, vorzugsweise kleiner gleich 35 ml, vorzugsweise kleiner gleich 30 ml, vorzugsweise kleiner gleich 15 ml, vorzugsweise kleiner gleich 7 ml.

Bezugszeichenliste

| 1 | Dialysierflüssigkeitsquelle |
| 2 | Dialysierflüssigkeitszufluss |
| 7 | Flusspumpe-Filterelement-Eingang (FPE) |
| 8 | Ventil Filterelement Eingang (VDE) |
| 9 | Bypass-Ventil (VBP) |
| 10 | Ventil Filterelement Ausgang (VDA) |
| 11 | Bypass-Leitung |
| 12 | Flusspumpe Dialysierflüssigkeitsausgang |
| 13 | Dialysierflüssigkeitsabflussleitung |
| 14 | Detektionsvorrichtung |
| 14a | Alternativposition Detektionsvorrichtung |
| 15 | Dialysierflüssigkeitsabfluss |
| 16 | Flussmesseinrichtung |
| 17 | arterielles Schlauchsystem |
| 18 | arterielle Blutpumpe (BPA) |
| 19 | Blutseite (BS) im Filterelement |
| 20 | Dialysierflüssigkeitsseite (DS) im Filterelement |
| 21 | venöses Schlauchsystem |
| 22 | Substitutionsflüssigkeitsquelle |
| 23 | Substitutionspumpe |
| 30 | Recheneinheit |
| 31 | User Interface |

**Patentansprüche**

1. Vorrichtung zur extrakorporalen Blutbehandlung umfassend

- ein Filterelement, das in eine blutseitige Kammer (19) und in eine dialysierflüssigkeitsseitige Kammer (20) geteilt ist, mit einem Zufluss (2) für Dialysierflüssigkeit zur dialysierflüssigkeitsseitigen Kammer (20) und einem Abfluss (13) für die Dialysierflüssigkeit aus der dialysierflüssigkeitsseitigen Kammer (20),
- eine Bypassleitung (11) und
- eine Detektionsvorrichtung (14), die im Betrieb kontinuierlich von aus dem Abfluss (13) abfliessender Dialysierflüssigkeit durchflossen wird und dazu ausgelegt ist, die zeitliche Konzentrationsänderung mindestens eines Abbauprodukts aus dem Blut des Patienten dialysierflüssigkeitsseitig zu erfassen und diese erfassten Werte bereit zu stellen, wobei
- die Detektionsvorrichtung (14) an einer Position stromab zum Abfluss (13) der Dialysierflüssigkeit aus der dialysierflüssigkeitsseitigen Kammer (20) des Filterelements direkt an der Fluidleitung angebracht ist,

**dadurch gekennzeichnet, dass**

die Detektionsvorrichtung (14) zwischen dem Abfluss (13) der Dialysierflüssigkeit aus der dialysierflüssigkeitsseitigen Kammer (20) des Filterelements und einem Anschluss der Bypass Leitung (11) angeordnet ist und das sich ergebende Fluidleitungs- und/oder Komponenten - Füllvolumen zwischen dem Abfluss (13) und der Detektionsvorrichtung (14) kleiner oder gleich 100 ml ist.

2. Vorrichtung zur extrakorporalen Blutbehandlung gemäß Anspruch 1, **gekennzeichnet durch** ein Vorrichtungsgehäuse (G) zur Aufnahme einer Mehrzahl Vorrichtungseigener Funktionselemente, innerhalb dessen auch die Detektionsvorrichtung (14) untergebracht ist und außerhalb dessen das Filterelement angeordnet ist, das über eine Gehäusedurchführung (L) mit der Detektionsvorrichtung (14) fluidverbunden ist.

3. Vorrichtung nach Anspruch 2, **dadurch gekennzeichnet, dass** die Detektionsvorrichtung an der Gehäusedurchführung (L) innenseitig oder außenseitig an einer die Gehäusedurchführung (L) aufweisenden Wand des Vorrichtungsgehäuses (G) platziert ist.

4. Vorrichtung zur extrakorporalen Blutbehandlung gemäß Anspruch 2 oder 3, **gekennzeichnet durch** einen Drucksensor (D), der stromauf zur Detektionsvorrichtung (14) im Bereich der Gehäusedurchführung (L) angeordnet ist.

5. Vorrichtung zur extrakorporalen Blutbehandlung gemäß einem der vorstehenden Ansprüche, **gekennzeichnet dadurch, dass** die Bypass - Leitung (11), den Einlass und den Auslass der dialysierflüssigkeitsseitigen Kammer (20) unter Kurzschließen des Filterelements wahlweise fluidverbindet.

6. Vorrichtung zur extrakorporalen Blutbehandlung gemäß einem der vorstehenden Ansprüche, **dadurch gekennzeichnet, dass** der innere Leitungsquerschnitt zumindest in dem Fluidleitungsabschnitt zwischen Filterelement und Detektionsvorrichtung (14) 3 - 7mm beträgt.

7. Vorrichtung zur extrakorporalen Blutbehandlung gemäß Anspruch 6, **dadurch gekennzeichnet, dass** der innere Leitungsquerschnitt zumindest in dem Fluidleitungsabschnitt zwischen Filterelement und Detektionsvorrichtung (14) konstant ist.

8. Vorrichtung zur extrakorporalen Blutbehandlung gemäß einem der vorstehenden Ansprüche, **dadurch gekennzeichnet, dass** die Detektionsvorrichtung (14) eine optische Sensorik in Form einer UV - Messvorrichtung hat.

9. Vorrichtung zur extrakorporalen Blutbehandlung gemäß Anspruch 6, **dadurch gekennzeichnet, dass** der innere Leitungsquerschnitt zumindest in dem Fluidleitungsabschnitt zwischen Filterelement und Detektionsvorrichtung (14) 5mm beträgt.

10. Vorrichtung zur extrakorporalen Blutbehandlung gemäß Anspruch 1, **dadurch gekennzeichnet, dass** das Fluidleitungs- und/oder Komponenten - Füllvolumen ausgehend vom Abfluss der verbrauchten Dialysierflüssigkeit aus dem Filterelement bis zur Detektionsvorrichtung (14) kleiner gleich 50 ml beträgt.

11. Vorrichtung zur extrakorporalen Blutbehandlung gemäß Anspruch 1, **dadurch gekennzeichnet, dass** das Fluidleitungs- und/oder Komponenten - Füllvolumen ausgehend vom Abfluss der verbrauchten Dialysierflüssigkeit aus dem Filterelement bis zur Detektionsvorrichtung (14) kleiner gleich 35 ml beträgt.

12. Vorrichtung zur extrakorporalen Blutbehandlung gemäß Anspruch 1, **dadurch gekennzeichnet, dass** das Fluidleitungs- und/oder Komponenten - Füllvolumen ausgehend vom Abfluss der verbrauchten Dialysierflüssigkeit aus dem Filterelement bis zur Detektionsvorrichtung (14) kleiner gleich 30 ml beträgt.

13. Vorrichtung zur extrakorporalen Blutbehandlung gemäß Anspruch 1, **dadurch gekennzeichnet, dass** das Fluidleitungs- und/oder Komponenten - Füllvolumen ausgehend vom Abfluss der verbrauchten Dialysierflüssigkeit aus dem Filterelement bis zur Detektionsvorrichtung (14) kleiner gleich 15 ml beträgt.

14. Vorrichtung zur extrakorporalen Blutbehandlung gemäß Anspruch 1, **dadurch gekennzeichnet, dass** das Fluidleitungs- und/oder Komponenten - Füllvolumen ausgehend vom Abfluss der verbrauchten Dialysierflüssigkeit aus dem Filterelement bis zur Detektionsvorrichtung (14) kleiner gleich 7 ml beträgt.

**Claims**

1. A device for extracorporeal blood treatment, comprising:

   - a filter element which is subdivided into a blood-sided chamber (19) and a dialysis liquid-side chamber (20) and comprises an inflow (2) for dialysis liquid to the dialysis liquid-side chamber (20) and an outflow (13) for the dialysis liquid from the dialysis liquid-side chamber (20),
   - a bypass line (11), and
   - a detection equipment (14), through which dialysis liquid flowing out of the outflow (13) consistently flows during operation, and adapted to detect the temporal concentration change of at least one metabolic product from the blood of the patient on the dialysis liquid side and to provide these measured values, wherein
   - the detection equipment (14) is disposed at a position downstream of the outflow (13) of the dialysis liquid from the dialysis liquid-side chamber (20) of the filter element directly at the fluid line,

   **characterized in that**
   the detection equipment (14) is disposed between the outflow (13) of the dialysis liquid from the dialysis liquid-side chamber (20) of the filter element and a terminal of the bypass line (11), and
   the resulting filling volume of the fluid line and/or of the components is less than or equal to 100 ml between the outflow (13) and the detection equipment (14).

2. The device for extracorporeal blood treatment according to claim 1, **characterized by** a device housing (G) for receiving a plurality of functional elements associated with the device, the detection equipment (14) being provided within said device housing, too, and the filter element being arranged outside of said device housing, the filter element being fluidly connected to the detection equipment (14) via a housing feedthrough (L).

3. The device according to claim 2, **characterized in that** the detection equipment is disposed on the housing feedthrough (L) at the inner side or outer side of a wall associated with the device housing (G) and comprising the housing feedthrough (L).

4. The device for extracorporeal blood treatment according to claim 2 or 3, **characterized by** a pressure sensor (D) which is arranged upstream of the detection equipment (14) in the vicinity of the housing feedthrough (L).

5. The device for extracorporeal blood treatment according to one of the preceding claims, **characterized in that** the bypass line (11) selectively establishes a fluid connection between the inlet and the outlet of the dialysis liquid-side chamber (20) while bypassing the filter element.

6. The device for extracorporeal blood treatment according to one of the preceding claims, **characterized in that** the line's inner cross-section amounts to 3 to 7mm at least in the fluid line section between the filter element and the detection equipment (14).

7. The device for extracorporeal blood treatment according to claim 6, **characterized in that** the line's inner cross-section is substantially constant at least in the fluid line section between the filter element and the detection equipment (14).

8. The device for extracorporeal blood treatment according to one of the preceding claims, **characterized in that** the detection equipment (14) comprises an optical sensor system in the form of a UV measuring device.

9. The device for extracorporeal blood treatment according to claim 6, **characterized in that** the line's inner cross-section amounts to 5mm at least in the fluid line section between the filter element and the detection equipment (14).

10. The device for extracorporeal blood treatment according to claim 1, **characterized in that** the filling volume of the fluid line and/or of the components starting from the outflow of the used dialysis liquid from the filter element to the detection equipment (14) is less than or equal to 50 ml.

11. The device for extracorporeal blood treatment according to claim 1, **characterized in that** the filling volume of the fluid line and/or of the components starting from the outflow of the used dialysis liquid from the filter element to the detection equipment (14) is less than or equal to 35 ml.

12. The device for extracorporeal blood treatment according to claim 1, **characterized in that** the filling volume of the fluid line and/or of the components starting from the outflow of the used dialysis liquid from the filter element to the detection equipment (14) is less than or equal to 30 ml.

13. The device for extracorporeal blood treatment according to claim 1, **characterized in that** the filling volume of the fluid line and/or of the components starting from the outflow of the used dialysis liquid from the filter element to the detection equipment (14) is less than or equal to 15 ml.

14. The device for extracorporeal blood treatment according to claim 1, **characterized in that** the filling volume of the fluid line and/or of the components starting from the outflow of the used dialysis liquid from the filter element to the detection equipment (14) is less than or equal to 7 ml.

**Revendications**

1. Dispositif de traitement extracorporel du sang comprenant :

 - un élément de filtrage qui est partagé en une chambre côté sang (19) et une chambre côté liquide de dialyse (20), avec une alimentation (2) pour le liquide de dialyse à la chambre côté liquide de dialyse (20) et une décharge (13) pour le liquide de dialyse de la chambre côté liquide de dialyse (20),
 - un conduit de dérivation (11) et
 - un dispositif de détection (14) qui est parcouru en service de manière continue par le liquide de dialyse évacué par la décharge (13) et est conçu de manière à saisir la modification de concentration dans le temps d'au moins un produit de dégradation du sang du patient côté liquide de dialyse et à affecter ces valeurs déterminées, dans lequel :
 - le dispositif de détection (14) est monté directement sur le conduit de fluide dans une position en aval de la décharge (13) du liquide de dialyse de la chambre côté liquide de dialyse (20) de l'élément de filtrage,

 **caractérisé en ce que** :

 le dispositif de détection (14) est agencé entre la décharge (13) du liquide de dialyse de la chambre côté liquide de dialyse (20) de l'élément de filtrage et une liaison du conduit de dérivation (11) et
 le volume de remplissage du conduit de fluide et/ou de composants qui apparaît entre la décharge (13) et le dispositif de détection (14) est inférieur ou égal à 100 ml.

2. Dispositif de traitement extracorporel du sang selon la revendication 1, **caractérisé par** un boîtier de dispositif (G) pour recevoir une pluralité d'éléments fonctionnels propres au dispositif, à l'intérieur desquels le dispositif de détection (14) est également inséré et à l'extérieur desquels est agencé l'élément de filtrage qui est en communication fluidique avec le dispositif de détection (14) via un passage de boîtier (L).

3. Dispositif selon la revendication 2, **caractérisé en**

**ce que** le dispositif de détection est placé sur le passage de boîtier (L) côté interne ou côté externe sur une paroi du boîtier (G) du dispositif présentant un passage de boîtier (L).

4. Dispositif de traitement extracorporel du sang selon la revendication 2 ou 3, **caractérisé par** un capteur de pression (D) qui est agencé en amont du dispositif de détection (14) dans la zone du passage (L) du boîtier.

5. Dispositif de traitement extracorporel du sang selon l'une quelconque des revendications précédentes, **caractérisé en ce que** le conduit de dérivation (11) met en communication fluidique l'entrée et la sortie de la chambre côté liquide de dialyse (20) en court-circuitant l'élément de filtrage facultativement.

6. Dispositif de traitement extracorporel du sang selon l'une quelconque des revendications précédentes, **caractérisé en ce que** la section transversale de conduit interne atteint 3 à 7 mm au moins dans la section de conduit de fluide entre l'élément de filtrage et le dispositif de détection (14).

7. Dispositif de traitement extracorporel du sang selon la revendication 6, **caractérisé en ce que** la section de conduit interne est constante au moins dans la section transversale de conduit de fluide entre l'élément de filtrage et le dispositif de détection (14).

8. Dispositif de traitement extracorporel du sang selon l'une quelconque des revendications précédentes, **caractérisé en ce que** le dispositif de détection (14) a un capteur optique sous la forme d'un dispositif de mesure aux UV.

9. Dispositif de traitement extracorporel du sang selon la revendication 6, **caractérisé en ce que** la section transversale de conduit interne atteint 5 mm au moins dans la section de conduit de fluide entre l'élément de filtrage et le dispositif de détection (14).

10. Dispositif de traitement extracorporel du sang selon la revendication 1, **caractérisé en ce que** :

le volume de remplissage du conduit de fluide et/ou de composants à partir de la décharge du liquide de dialyse consommé de l'élément de filtrage jusqu'au dispositif de détection (14) est inférieur ou égal à 50 ml.

11. Dispositif de traitement extracorporel du sang selon la revendication 1, **caractérisé en ce que** :

le volume de remplissage de conduit de fluide et/ou de composants à partir de la décharge du liquide de dialyse consommé de l'élément de filtrage jusqu'au dispositif de détection (14) est inférieur ou égal à 35 ml.

12. Dispositif de traitement extracorporel du sang selon la revendication 1, **caractérisé en ce que** :

le volume de remplissage de conduit de fluide et/ou de composants à partir de la décharge du liquide de dialyse consommé de l'élément de filtrage jusqu'au dispositif de détection (14) est inférieur ou égal à 30 ml.

13. Dispositif de traitement extracorporel du sang selon la revendication 1, **caractérisé en ce que** :

le volume de remplissage de conduit de fluide et/ou de composants à partir de la décharge du liquide de dialyse consommé de l'élément de filtrage jusqu'au dispositif de détection (14) est inférieur ou égal à 15 ml.

14. Dispositif de traitement extracorporel du sang selon la revendication 1, **caractérisé en ce que** :

le volume de remplissage de conduit de fluide et/ou de composants à partir de la décharge du liquide de dialyse consommé de l'élément de filtrage jusqu'au dispositif de détection (14) est inférieur ou égal à 7 ml.

Figur 1

Figur 2

Figur 3

Figur 4

Figur 5

Figur 6

EP 2 799 097 B1

Figur 7

Figur 8

Figur 9

23

Figur 10

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20110144459 A1
- WO 0038761 A2
- EP 1342479 A1
- WO 9744072 A1
- EP 0898974 A2
- EP 0898974 A
- FR 2801794 A1
- EP 1083948 B1
- DE 102011008482 A1
- EP 2397167 A1

**In der Beschreibung aufgeführte Nicht-Patentliteratur**

- Haemodialysis Treatment monitored on-line by ultra violet absorbance. Linköping University Medical Dissertation, 2006
- **CALIA ; CALIA D. ; DI FRANCESCO F. ; FUOCO R. ; GHIMENTI S. ; KANAKI A. ; ONOR M. ; TOGNOTTI D. ; DONADIO C. et al.** bestätigt (Monitoring Urea, Creatinine and ß2-Microglobulin Concentrations in Spent Dialysate by Spectrophotometric and Spectrofluorimetric Measurements. *52nd National Congress of the Italian Society of Nephrology,* 21. September 2011
- **B. J. KIRBY.** Micro- and Nanoscale Fluid Mechanics. Cambridge University Press, 2010